(19) Europäisches Patentamt
European Patent Office
Office européen des brevets

(11) Publication number: **0 541 252 A2**

(12) **EUROPEAN PATENT APPLICATION**

(21) Application number: **92309243.1**

(51) Int. Cl.[5]: **C07C 213/02**

(22) Date of filing: **09.10.92**

(30) Priority: **04.11.91 US 787209**

(43) Date of publication of application: **12.05.93 Bulletin 93/19**

(84) Designated Contracting States: **DE FR GB**

(71) Applicant: **TEXACO CHEMICAL COMPANY**
**3040 Post Oak Boulevard**
**Houston, Texas 77056(US)**

(72) Inventor: **Hollingsworth, Donald Ray**
**2000 Delwood Court**
**Austin, Texas 78723(US)**
Inventor: **Zimmerman, Robert LeRoy**
**4202 Cordova**
**Austin, Texas 78759(US)**

(74) Representative: **Brock, Peter William et al**
**UROUHART-DYKES & LORD 91 Wimpole Street**
**London W1M 8AH (GB)**

# (54) Catalytic method for the reductive amination of poly(oxytetramethylene)diacetates.

(57) Poly(oxytetramethylene) diamines having the formula:

$$H_2N-CH_2-CH_2-CH_2-CH_2-[-o-CH_2-CH_2-CH_2-CH_2-]_n-o-CH_2-CH_2-CH_2-CH_2-NH_2 \quad (I)$$

wherein n represents 0 or a positive number up to 50 which can be prepared with good yield and selectivity, by contacting a poly(oxytetramethylene) diacetate having the formula:

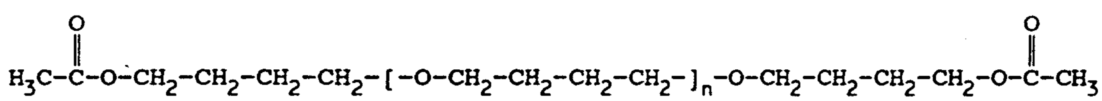

(II)

with hydrogen and ammonia in the presence of a catalyst comprising, on an oxide-free basis, 70 to 78 wt.% of nickel, 20 to 25 wt.% of copper, 0.5 to 5 wt.% of chromium and 1 to 5 wt.% of molybdenum.

EP 0 541 252 A2

This invention relates to a method for the catalytic reductive amination of a poly(oxytetramethylene) diacetate in order to provide the corresponding poly(oxytetramethylene) diamine, in high yield and with good selectivity.

Still more particularly, this invention relates to a process wherein a poly(oxytetramethylene) diacetate is reacted with excess ammonia and hydrogen in the presence of a nickel, copper, chromium, molybdenum catalyst in order to provide a reaction product comprising the poly(oxytetramethylene) diamine corresponding to the poly(oxytetramethylene) glycol feedstock.

Methods for the preparation and use of poly(oxytetramethylene) glycols are disclosed in US-A-3824198, US-A-3824219 and US-A-3824220. Other Patents disclosing methods for making and using **poly(oxytetramethylene) glycols** and poly(oxytetramethylene) diamines include US-A-3436359 and US-A-4833213.

US-A-4618717 is directed to a method for reductively aminating ethylene glycol monoalkyl ethers in order to provide the corresponding primary amines using a catalyst composed of 50 to 90 wt.% of nickel, 10 to 50 wt.% of copper and 0.5 to 5 wt.% of an oxide of chromium, iron, titanium, thorium, zirconium, manganese, magnesium or zinc. US-A-4766245 discloses a method for reductively aminating polyoxyalkylene diols and triols in the presence of a Raney nickel/aluminium catalyst.

US-A-5003107 discloses a method for the preparation of poly(oxytetramethylene) diamines by the catalytic reductive amination of a poly(oxytetramethylene) glycol feedstock in the presence of hydrogen and ammonia using a hydrogenation/dehydrogenation catalyst composed of, on an oxide-free basis, 70 to 75 wt.% of nickel, 20 to 25 wt.% of copper, 0.5 to 5 wt.% of chromium and 1 to 5 wt.% of molybdenum. This process suffers from the disadvantageous need to convert tetrahydrofuran or an oligomer thereof into a glycol.

This invention relates to a method for preparing poly(oxytetramethylene) diamines. Poly(oxytetramethylene) diamines are useful for the preparation of polyamide and polyurea elastomers. The poly(oxytetramethylene) diamines have previously been prepared from the corresponding poly(oxytetramethylene) glycols, commonly referred to as polytetrahydrofuran glycols. An example is the method disclosed in the above-mentioned US-A-5003107 which requires the preparation of an intermediate glycol derivative of tetrahydrofuran. In contrast, in accordance with the present invention, poly(oxytetramethylene) diacetate resulting from the polymerization of tetrahydrofuran is converted into the corresponding poly(oxytetramethylene) diamines by catalytic reductive amination.

More specifically, the present invention provides a method for the preparation of a poly(oxytetramethylene) diamine having the formula:

$$H_2N-CH_2-CH_2-CH_2-CH_2-[-o-CH_2-CH_2-CH_2-CH_2-]_n-o-CH_2-CH_2-CH_2-CH_2-NH_2 \quad (I)$$

wherein n represents 0 or a positive number up to 50 which comprises contacting a poly(oxytetramethylene) diacetate having the formula:

$$H_3C-\overset{\overset{O}{\|}}{C}-O-CH_2-CH_2-CH_2-CH_2-[-O-CH_2-CH_2-CH_2-CH_2-]_n-O-CH_2-CH_2-CH_2-CH_2-O-\overset{\overset{O}{\|}}{C}-CH_3$$

(II)

with hydrogen and ammonia in the presence of a catalyst comprising, on an oxide-free basis, 70 to 78 wt.% of nickel, 20 to 25 wt.% of copper, 0.5 to 5 wt.% of chromium and 1 to 5 wt.% of molybdenum.

The catalyst used according to the present invention may suitably be a powdered catalyst, if the reaction is to be conducted in an autoclave on a batch basis, or pelleted catalyst, if the reaction is to be conducted on a continuous basis in a continuous reactor.

The catalyst is suitably a catalyst of the type disclosed in US-A-315115.

In accordance with the present invention, the poly(oxytetramethylene) diacetate feedstock is substantially quantitatively converted into the corresponding poly(oxytetramethylene) diamine with excellent yields and selectivities.

An especially preferred catalyst composition for use in the present invention contains from 70 to 75 wt.% of nickel, 20 to 25 wt.% of copper, 0.5 to 3 wt.% of chromium, and 1 to 3 wt.% of molybdenum.

The reductive amination reaction of the present invention is suitably conducted at a temperature of 150 to 220°C and a pressure of 0.75 to 70 MPa, more preferably, 0.75 to 21 MPa.

The reductive amination is conducted in the presence of ammonia. Suitably, from 1 to 300 moles of ammonia, more preferably 10 to 150 moles of ammonia, are employed per mole of ploy(oxytetramethylene) diacetate.

The reaction is conducted in the presence of added hydrogen. The amount of added hydrogen used may be 0.1 to 10 mole, more preferably from 0.5 to 8.0 mole, of hydrogen per mole of poly-(oxytetramethylene) diacetate.

The process of the present invention may be conducted batch-wise, using an autoclave containing powdered catalyst, in which case the residence time is suitably from 0.5 to 5 hours.

More preferably, the reaction is conducted on a continuous basis using a bed of pelleted catalyst through which the hydrogen, ammonia and poly(oxytetramethylene) diacetate are passed. When the reaction is conducted on a continuous basis, the poly(oxytetramethylene) diacetate is suitably charged to the catalyst bed at the rate of about 0.5 grams per hour of poly(oxytetramethylene) diacetate per $cm^3$ of catalyst.

The reaction mixture formed as a result of the reductive amination of the poly(oxytetramethylene) diacetate may be recovered and fractionated in any suitable manner, such as by fractional distillation, to obtain unreacted feed components, by-products and the desired poly(oxytetramethylene) diamine reaction product. Conversions of 90 wt.% or more, and selectivities of 90% or more, are obtainable with the process of the present invention, such that only minor quantities of unreacted feedstock and by-products are present in the reaction mixture.

## EXAMPLES

The present invention will be further illustrated by the following specific Examples.

The nickel, copper, chromium, molybdenum catalyst used in conducting the batch and continuous experiments reported in the Examples comprised 75 wt.% of nickel, 21 wt.% of copper, 2 wt.% of chromium and 2 wt.% of molybdenum.

### (A) Preparation of Poly(tetrahydrofuran) Diacetate

Tetrahydrofuran or THF (500 g, 6.9 mol), dried over 4A molecular sieves, was added to Clay 124 from Engelhard (25 g) followed by distilled acetic anhydride (83 g, 0.81 mol). This mixture was stirred at 30°C for 48 hours. After this reaction period, the sample was stripped of light materials and the THF by heating to 75°C under reduced pressure (3.3 kPa). The viscous oil was placed under minimum vacuum (13 Pa) and 80°C for 1 hour to give 401 g of product (69% conversion); SAP # (Saponification No.) 3.23 (meq/g), 604 mw by gel permeation chromatography, 6.87 acidity (mgKOH/g), and 0.03 wt.% water.

### (B) Amination of Poly(tetrahydrofuran) Diacetate

A 1-litre autoclave was charged with 100 g of the diacetate from the above example (A), 50 g of the Ni-Cu-Co-MO catalyst, and 150 g of ammonia. The autoclave was then pressurized to 2.51 MPa (350 psig) with hydrogen and heated over a 85 minute period to 220°C. It was held at this temperature for 3 hours. The reaction mixture was then cooled and vented. The catalyst was removed by filtration, and the product was stripped at reduced pressure. It had the following analysis:

| | | |
|---|---|---|
| Total acet. | 0.945 meq/g | |
| Total amine | 0.843 meq/g | 89% amination |
| Primary amine | 0.642 meq/g | 76% of amines |

### (C) Comparison Example

A 1-litre autoclave was charged with 299 g of a 2000 molecular weight polytetrahydrofuran polyol, 48 g of the Ni-Cu-Co-Mo catalyst, and 90 g of ammonia. The autoclave was then pressurized to 2.51 MPa (350 psig) with hydrogen and heated over a 46 minute period to 220°C. It was held at that temperature for 3 hours. The reaction mixture was then cooled and vented. The catalyst was removed by filtration and the

product stripped at reduced pressure. The product had the following analysis:

| | | |
|---|---|---|
| Total acet. | 0.847 meq/g | |
| Total amine | 0.688 meq/g | 81% amination |
| Primary amine | 0.556 meq/g | 81% of amines |

## Claims

1. A method for the preparation of a poly(oxytetramethylene) diamine having the formula:

   $H_2N-CH_2-CH_2-CH_2-CH_2-[-o-CH_2-CH_2-CH_2-CH_2-]_n-o-CH_2-CH_2-CH_2-CH_2-NH_2$ (I)

   wherein n represents 0 or a positive number up to 50 which comprises contacting a poly-(oxytetramethylene) diacetate having the formula:

   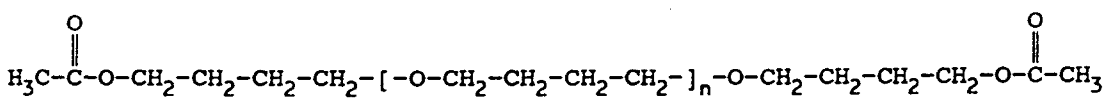

   (II)

   with hydrogen and ammonia in the presence of a catalyst comprising, on an oxide-free basis, 70 to 78 wt.% of nickel, 20 to 25 wt.% of copper, 0.5 to 5 wt.% of chromium and 1 to 5 wt.% of molybdenum.

2. A method according to Claim 1 characterised in that the catalyst comprises, on an oxide-free basis, 70 to 75 wt.% of nickel, 20 to 25 wt.% of copper, 0.5 to 3 wt.% of chromium and 1 to 3 wt.% of molybdenum.

3. A method according to Claim 1 or 2 characterised in that the poly(oxytetramethylene) diacetate is reacted at a temperature of 150 to 220° C and a pressure of 0.75 to 70 MPa.

4. A method according to Claim 3 characterised in that the pressure is from 0.75 to 21 MPa.

5. A method according to any one of Claims 1 to 4 characterised in that the reaction is conducted continuously by passing the poly(oxytetramethylene) diacetate, ammonia and hydrogen through a bed of the catalyst in pelleted form at a space velocity of 0.1 to 20 grams per hour of said poly-(oxytetramethylene) diacetate, per $cm^3$ of catalyst.

6. A method according to any one of Claims 1 to 5 characterised in that from 1 to 300 moles of ammonia are employed per mole of poly(oxytetramethylene) diacetate.

7. A method according to Claim 6 characterised in that 10 to 150 moles of ammonia are employed per mole of poly(oxytetramethylene) diacetate.

8. A method according to any one of Claims 1 to 7 characterised in that 0.1 to 10 moles of hydrogen are employed per mole of poly(oxytetramethylene) diacetate.

9. A method according to Claim 8 characterised in that 0.5 to 8 moles of hydrogen are employed per mole of poly(oxytetramethylene) diacetate.